# EUROPÄISCHE PATENTSCHRIFT

(11) **EP 2 628 787 B1**
(45) Veröffentlichungstag und Bekanntmachung des Hinweises auf die Patenterteilung: **21.05.2014**
(21) Anmeldenummer: 13000277.7
(22) Anmeldetag: 19.01.2013
(51) Int. Cl.: C12M 1/00, C12M 1/26

(54) **Probeentnahmevorrichtung und Verfahren zur Entnahme einer Probe aus einem Bioreaktor**
Device and method for taking a sample from a bioreactor
Dispositif de prélèvement d'échantillon et procédé de prélèvement d'un échantillon à partir d'un bioréacteur

(30) Priorität: 16.02.2012 DE 102012003113
(43) Veröffentlichungstag der Anmeldung: 21.08.2013
(73) Patentinhaber: Forschungszentrum Jülich GmbH, 52425 Jülich (DE)
(72) Erfinder: Paczia, Nicole, 40468 Düsseldorf (DE); Mottyll, Stephan, 44651 Herne (DE); Von Lieres, Eric, 50733 Köln (DE)

(56) Entgegenhaltungen:
- WO-A2-2004/087859
- DE-A1- 4 407 439
- DE-A1-102006 019 242
- DE-U1- 20 316 936
- US-A- 6 071 727

## Beschreibung

Die Erfindung betrifft eine Probeentnahmevorrichtung sowie ein Verfahren zur Entnahme einer Probe aus einem Bioreaktor.

In der weißen Biotechnologie werden chemische Wertstoffe unter anderem mit Mikroorganismen oder mit tierischen oder pflanzlichen Zellkulturen hergestellt. Um die Stoffwechselleistung der dafür eingesetzten Zellen und die für deren Kultivierung verwendeten biotechnologischen Prozesse zu untersuchen, werden stationäre und nichtstationäre Experimente in Bioreaktoren durchgeführt. Die entsprechenden Zellen weisen einen Stoffwechselhaushalt auf, der sensibel auf Störungen reagiert. Diese Störungen können bewusst herbeigeführt werden, in dem beispielsweise die zugeführten Mengen an Nährstoff, Sauerstoff oder Cosubstrat verändert werden, um die Reaktion der Mikroorganismen auf diese Veränderungen zu untersuchen. Es können aber auch nicht gewollte Störungen auftreten, die sich einstellen, wenn die Zellen in der Reaktorflüssigkeit nicht gewünschten Bedingungen ausgesetzt werden. Diese Störungen beeinflussen ebenfalls die Stoffwechselabläufe in den Zellen. Bei Stoffwechselexperimenten werden dem Bioreaktor zu definierten Zeiten Proben entnommen und für Messungen der intrazellulären und extrazellulären Stoffkonzentration vorbereitet. Ein wesentlicher Bestandteil dieser Vorbereitung ist eine schnelle Abkühlung der Proben, die der Inaktivierung des Zellstoffwechsels dient, da dieser sonst die Ergebnisse der anschließenden Messungen verfälschen würde.

Die nach dem Stand der Techniken verwendeten Probeentnahmevorrichtungen und Verfahren zur Probeentnahme basieren auf einer sequentiellen Vorgehensweise bei der die Probe zunächst dem Bioreaktor entnommen und anschließend in einer separaten Einrichtung gekühlt wird. Eine derartige Probeentnahmevorrichtung ist in der Veröffentlichung "New Bioreactor-Coupled Rapid Stopped-Flow Sampling Technique for Measurements of Metabolic Dynamics on a Subsecond Time Scale" von Stefan Buziol et al. in Biotechnology and Bioengeneering Vol. 80, No. 6, (Dezember 20, 2002) beschrieben. Bei der in dem Artikel beschriebenen Probeentnahmevorrichtung kommt ein Ventilsystem zum Einsatz, das geringe Totvolumina aufweist und eine genaue Dosierung der Probe erlaubt. Hinter dem Ventil werden die gezogenen Proben über eine gewisse Strecke transportiert und erst anschließend gekühlt, zum Beispiel durch Einspritzen in ein Gefäß, das eine vorgelegte Kühlflüssigkeit enthält. Dieser Prozess wird auch als Quenching bezeichnet. In der Veröffentlichung "Rapid sampling devices for metabolic engeneering applications" in Appl. Microbiol. Biotechnol. (2009) 83:199-208 von F. Schädel und E. Franco-Lara werden Vorrichtungen beschrieben, mit denen Reaktorflüssigkeiten entnommen und gekühlt werden können. Typische Kühlmittel für diese Zwecke werden von Walter M. van Gulik in dem Artikel "Fast sampling for quantitative microbial metabolomics" in Current Opinion in Biotechnology 2010, 21: 27-34 dargestellt.

DE 44 07 439 offenbart ein Verfahren zur Untersuchung des zeitlichen Ablaufs von Bioreaktionen in einem Reaktionsraum durch aufeinanderfolgende Entnahme biologischer Proben daraus unter Zumischung von Inaktivierungsmittel am Probennahmeort mit einer als Doppelrohr ausgebildeten Probennahmesonde

WO 2004/087859 offenbart ein Probennahmesystem für fluide Proben. Derartige Probennahmesysteme werden insbesondere benötigt, wenn eine Probe aus einer Flüssigkeit mit einer Simultan-Inaktivierung der Probe entnommen werden soll. Ziel einer derartigen schnellen Probenahme ist es dabei, trotz hoher Umsatzraten einer in der Flüssigkeit stattfindenden chemischen oder biologischen Reaktion ein repräsentatives Abbild der in der Flüssigkeit zum Zeitpunkt der Probenahme vorliegenden Konzentrationen zu erhalten.

US 6 071 727 offenbart die Verwendung von Diskusartigen Mischvorrichtungen in einer Röhre als Füllkörper für zu mischende Flüssigkeitsströme.

Die nach dem Stand der Technik eingesetzten Verfahren und Probeentnahmevorrichtungen sind trotz der geringen Totvolumina ungenau und führen zu gekühlten Proben von Zellen, deren intrazelluläre Stoffkonzentrationen sich von denen der sich im Bioreaktor befindlichen Zellen unterscheiden, so dass der Stoffwechselzustand der sich im Bioreaktor befindlichen Zellen bei der Messung der Konzentrationen dieser Proben nicht realistisch abgebildet wird. Insbesondere führen sie dazu, dass zwischen der Entnahme von Zellen aus einem Reaktor und der Kühlung dieser Zellen eine undefinierte Zeit vergeht. Da die Stoffwechselabläufe in den Zellen erst durch die Kühlung angehalten werden, führt dieser zeitliche Versatz dazu, dass sich die intrazellulären Stoffwechselkonzentrationen der Zellen in der Probe von denen in den Zellen unterscheiden. Eine Bestimmung des Stoffwechselzustandes der sich im Bioreaktor befindlichen Zellen ist daher nicht möglich.

Es ist daher die Aufgabe der Erfindung eine Probeentnahmevorrichtung und ein Verfahren zu schaffen, mit denen eine schnelle und gleichmäßige Kühlung der aus dem Bioreaktor entnommenen Probe durchgeführt werden kann, bei der die Stoffkonzentration in den sich in der Probe befindenden Zellen besser erhalten bleibt.

Ausgehend vom Oberbegriff des Anspruchs 1 und des nebengeordneten Anspruchs wird die Aufgabe gelöst mit den im kennzeichneten Teil des Anspruchs 1 und des nebengeordneten Anspruchs angegebenen Merkmalen.

Mit der erfindungsgemäßen Probeentnahmevorrichtung und dem erfindungsgemäßen Verfahren ist es nunmehr möglich, die Proben innerhalb von höchstens 200 ms in jedem Punkt des Probevolumens auf mindestens -20° C herabzukühlen. Der Stoffwechselzustand der gekühlten Zellen entspricht besser dem der sich im Bioreaktor enthaltenden Zellen. Die Probeentnahmevorrichtung ist daher auch eine Kühlvorrichtung.

Vorteilhafte Weiterbildungen der Erfindung sind in den Unteransprüchen angegeben.

Die Figuren zeigen die erfindungsgemäße Probeentnahmevorrichtung in schematischer Form.

Es zeigt
- Fig. 1:: Den Aufbau der erfindungsgemäßen Probeentnahmevorrichtung in geöffnetem Zustand
- Fig. 2:: Den Aufbau der erfindungsgemäßen Probeentnahmevorrichtung in geschlossenem Zustand
- Fig. 3:: Einen Ausschnitt der erfindungsgemäßen Probeentnahmevorrichtung mit einer Mischvorrichtung.
- Fig. 4a bis 4d:: Graustufenbilder in denen die Durchmischung entlang des Strömungsverlaufes dargestellt ist.
- Fig. 5: Eine Darstellung des Stempels
- Fig. 5a:: Einen Schnitt A-A des Stempels
- Fig. 5b:: Einen Schnitt B-B des Stempels
- Fig. 5c:: Eine Aufsicht auf die Stirnseite des Stempels
- Fig. 6:: Einen Schnitt durch die Probeentnahmevorrichtung
- Fig. 6a:: Eine Aufsicht auf die Probeentnahmevorrichtung von hinten
- Fig. 6b:: Eine perspektivische Ansicht der Probeentnahmevorrichtung von vorne
- Fig. 6c:: Eine perspektivische Ansicht der Probeentnahmevorrichtung von hinten
- Fig. 6d:: Eine Seitenansicht der Probeentnahmevorrichtung
- Fig. 7:: Eine Aufsicht auf die einem Reaktorinnenraum zugewandten Seite der Probeentnahmevorrichtung
- Fig. 7a:: Eine Seitenansicht der Probeentnahmevorrichtung
- Fig. 7b:: Einen Querschnitt C-C der Probeentnahmevorrichtung
- Fig. 7c:: Eine dreidimensionale Darstellung eines Schnitts durch die Probeentnahmevorrichtung
- Fig. 7d:: Eine plastische Darstellung der Probeentnahmevorrichtung
- Fig. 8a-c:: Verschiedene plastische Ansichten der Probeentnahmevorrichtung

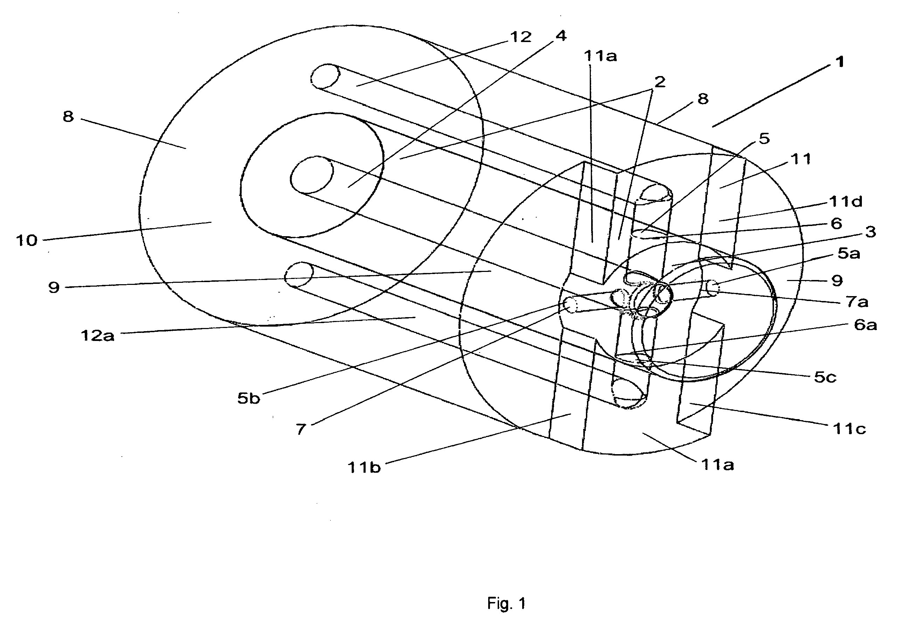

In Figur 1 wird eine Probeentnahmevorrichtung 1 dargestellt, die einen Stempel 2 mit einem Stempelboden 3 aufweist, welcher eine Mischvorrichtung 4 umfasst. Der Stempel 2 besitzt an der einem Stempelboden 3 zugewandten Seite Öffnungen 5, 5a, 5b, 5c, die über Kühlmittelzuleitungen 6, 6a und Reaktorflüssigkeitszuleitungen 7, 7a mit der Mischvorrichtung verbunden sind. Die Mischvorrichtung 4 hat die Form einer Röhre und enthält Füllkörper, die in Figur 1 der Übersichtlichkeit halber nicht dargestellt sind. Der Stempel 2 mit der Mischvorrichtung 4, dem Stempelboden 3, sowie den Kühlmittelzuleitungen 6, 6a und Reaktorflüssigkeitszuleitungen 7, 7a sind in der Vorrichtung nach Figur 1 in einem Stück gefertigt. Der Stempelboden 3 schließt die Mischvorrichtung 4 am Ende mit den Kühlmittelzuleitungen 6, 6a und Reaktorflüssigkeitszuleitungen 7, 7a ab. Der Stempel 2 mit der Mischvorrichtung 4, dem Stempelboden 3, sowie den Kühlmittelzuleitungen 6, 6a und Reaktorflüssigkeitszuleitungen 7, 7a befindet sich in einer Hülse 8, welche an ihrem Kopfende eine Wand 9 besitzt. Eine Führung 10 ermöglicht das Einführen des Stempels 2 mit der Mischvorrichtung 4 in die Hülse 8. Die Wände 11, 11a, 11b, 11c, 11d schirmen den Innenraum der Hülse 8 gegen den Innenraum eines Reaktors ab, an den die Probeentnahmevorrichtung 1 angebracht werden kann. In der Hülse 8 befinden sich Zuleitungen 12, 12a für Kühlmittel, die in die Öffnungen 5, 5c des Stempels 2 münden und die dann in die Kühlmittelzuleitungen 6, 6a münden.

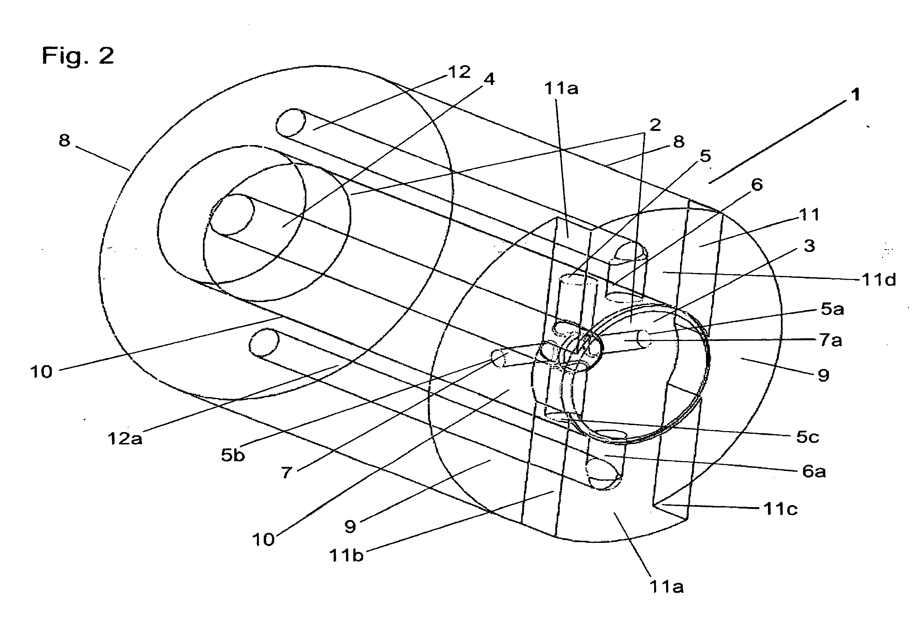

In Figur 2 haben gleiche Vorrichtungsmerkmale dieselben Bezugszeichen. Sie zeigt eine Darstellung der Probeentnahmevorrichtung, bei der der Stempel 2 gegenüber der Darstellung in Figur 1 etwas herausgezogen dargestellt ist. Damit befindet sich die Probeentnahmevorrichtung 1 in geschlossenem Zustand.

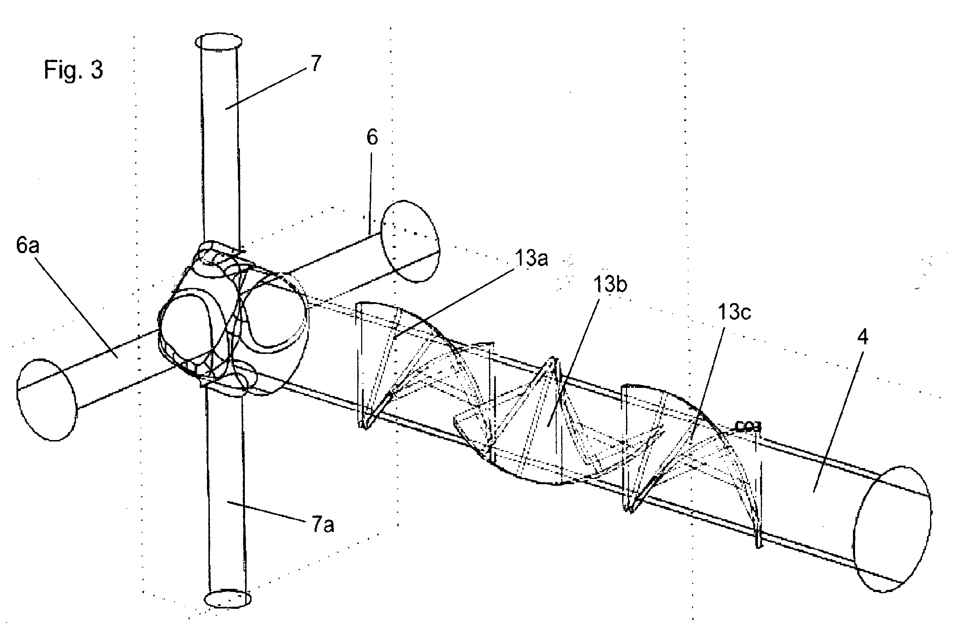

In Figur 3 wird eine Mischvorrichtung 1 in Form einer Mischröhre dargestellt, die mit Füllkörpern 13, 13a, 13b ... beschickt sind. Die Füllkörper 13, 13a, 13b ... haben die Form von Flächenelementen, welche entlang des Durchmessers der Mischröhre an einer Kante aufgespannt sind und entlang der Rotationsachse der Mischröhre verdreht sind und mit der, der ersten Kante gegenüberliegenden Kante entlang des Durchmessers der Mischröhre aufgespannt ist.

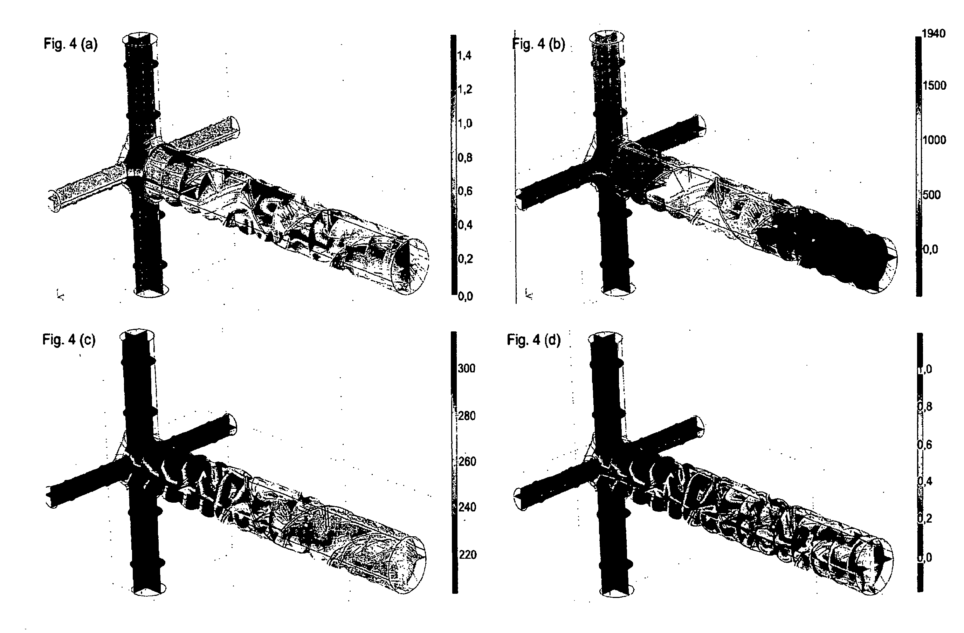

In Figur 4a, 4b, 4c und 4d ist der Vorrichtungsbestandteil aus Figur 3 mit Graustufen ausgefüllt, die den Mischungsgrad einer die Mischvorrichtung 4 durchströmende Flüssigkeit angeben. Dabei zeigt die Graphik (a) die Geschwindigkeit [m/s], Graphik (b) den Druck [Pa], Graphik (c) die Temperatur [K] und Graphik (d) die Konzentration der Kulturflüssigkeit [mol/mol].

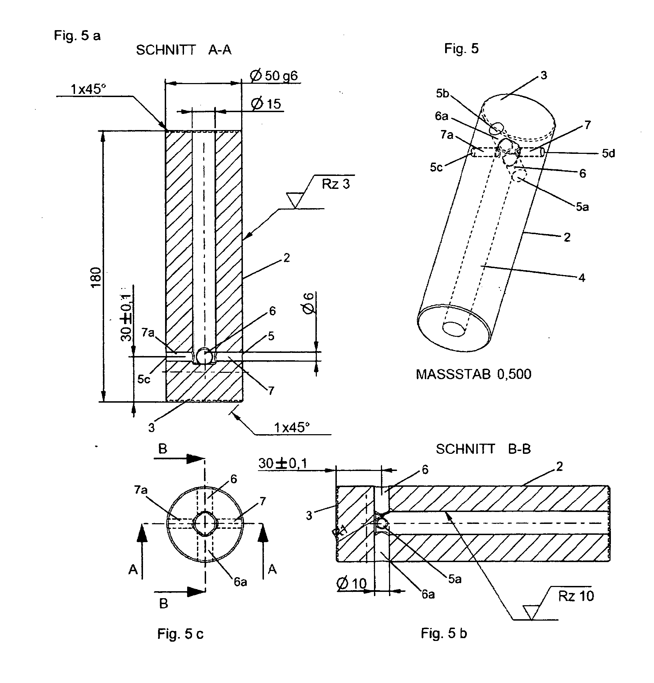

Die Figuren 5, 5a, 5b, 5c zeigen den Stempel 2 mit der Mischvorrichtung 4, den Kühlmittelzuleitungen 6, 6a und Reaktorflüssigkeitszuleitungen 7, 7a und den Öffnungen 5, 5a, 5b, 5c.

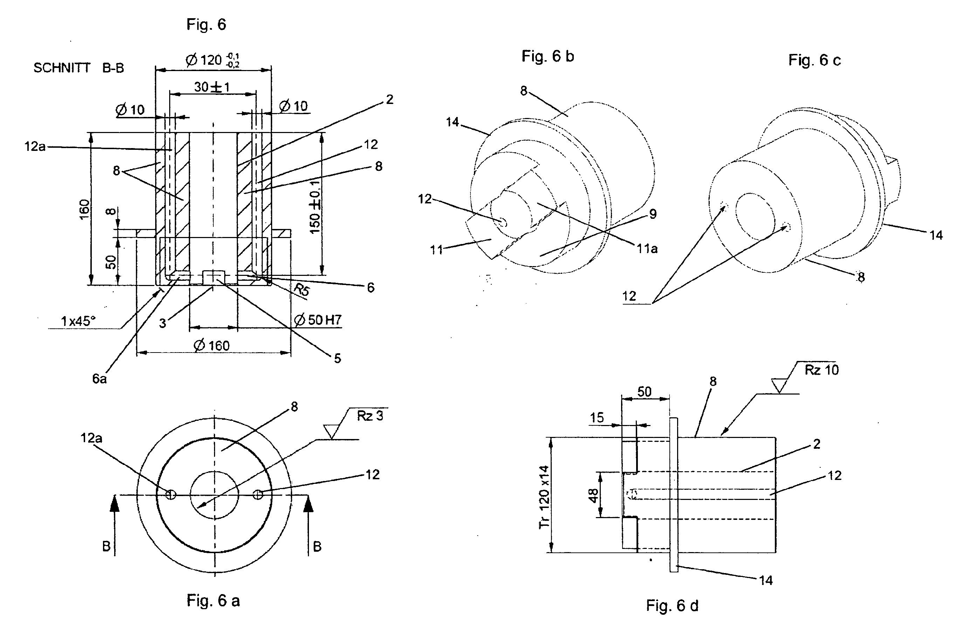

In den Figuren 6, 6a haben die Vorrichtungsmerkmale dieselben Bezugszeichen, wie in Figur 1.

Figur 6b und 6c zeigen eine Vorrichtung in der gleiche Vorrichtungsmerkmale dieselben Bezugszeichen haben. In Figuren 6b, 6c ist ein Ring 14 dargestellt, mit dem die Vorrichtung an einer Reaktorwand positioniert werden kann.

In Figur 6d haben gleiche Vorrichtungsmerkmale dieselben Bezugszeichen.

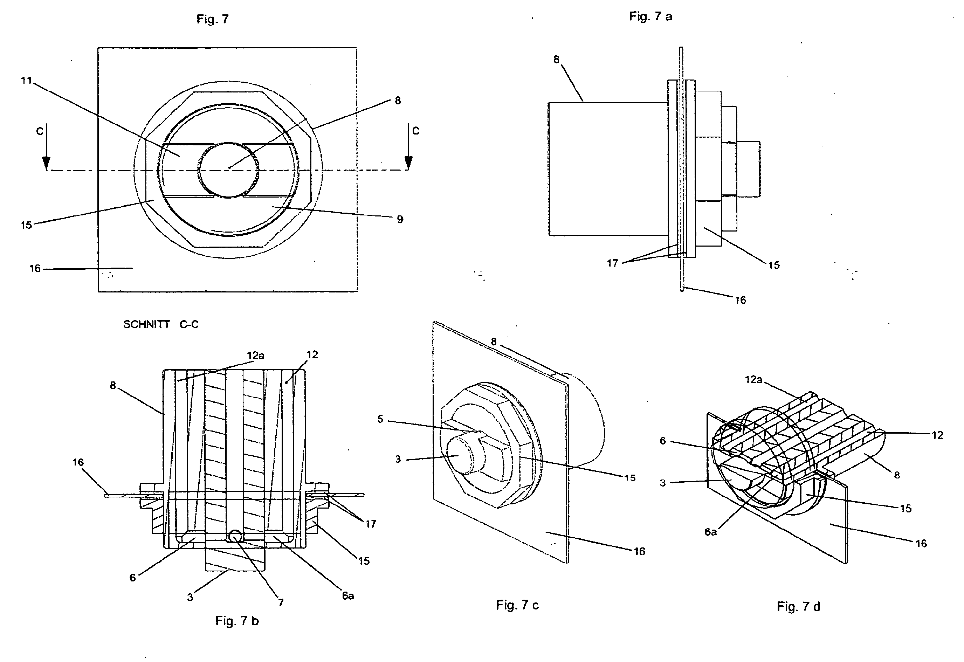

In Figur 7 ist eine Schraube 15 als Befestigungsmittel dargestellt. Das Bezugszeichen 16 bezeichnet eine Reaktorwand.

Figur 7a zeigt eine Seitenansicht der Figur 7 mit Dichtringen 17.

Figur 7b zeigt einen Schnitt.

In den Figuren 7c und 7d haben die gleichen Vorrichtungsmerkmale dieselben Bezugszeichen.

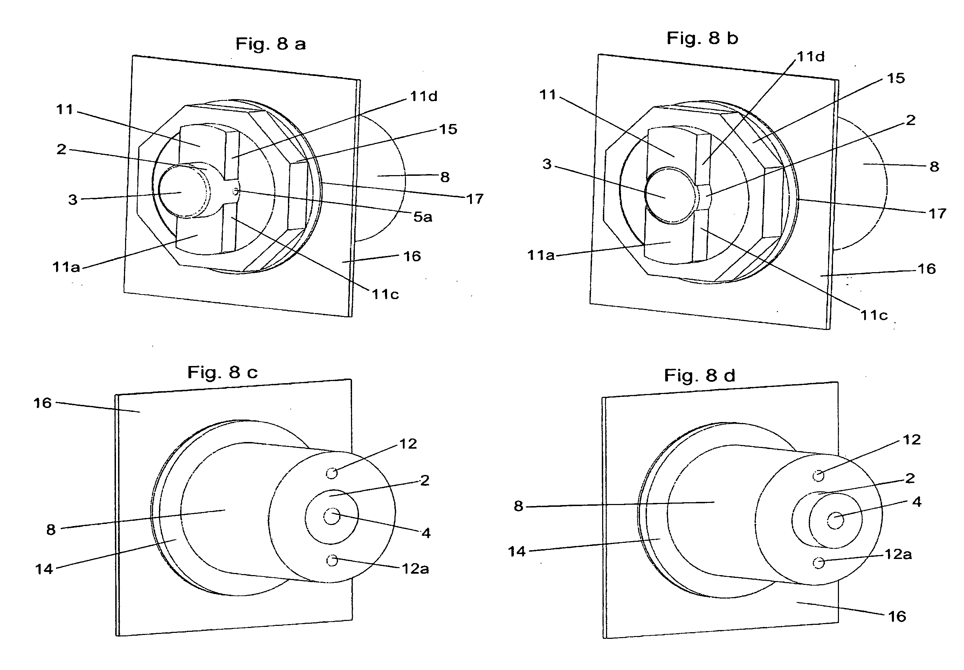

Die Figuren 8a, 8b, 8c und 8d zeigen die Vorrichtung ebenfalls in geöffnetem und geschlossenem Zustand.

Im Folgenden soll die Erfindung in ihrer allgemeinen Form erläutert werden.

Die erfindungsgemäße Probeentnahmevorrichtung 1 hat die Funktion eines Entnahmeventils für Reaktorflüssigkeit und dient der schnellen Kühlung der Reaktorflüssigkeit bei der Probeentnahme. Die Kühlung ist so schnell, dass die biochemischen Reaktionen, die sich im Inneren einer Zelle, die sich in der entnommenen Reaktorflüssigkeit befindet, gequencht werden, so dass sich die Stoffkonzentrationen im Inneren der Zelle und in der entnommenen Reaktorflüssigkeit nach der Entnahme kaum oder idealer weise nicht verändern. Die Probeentnahmevorrichtung 1 ist daher auch eine Kühlvorrichtung.

In einer bevorzugten Ausführungsform ist die erfindungsgemäße Probeentnahmevorrichtung 1 aus massiven Teilen gebaut.

In ihr ist der Stempel 2 als einteiliger Block ausgebildet, in dem die Mischvorrichtung 4, die Kühlmittelzuleitungen 6, 6a, die Reaktorflüssigkeitszuleitungen 7, 7a sowie die Öffnungen 5, 5a, 5b, 5c als Bohrungen ausgebildet sind. Die Mischvorrichtung 4 verläuft als Bohrung von einem Ende des Stempels 2 und endet am Stempelboden 3, der sie vom Innenraum des Reaktors trennt. Die Kühlmittelzuleitungen 6, 6a, die Reaktorflüssigkeitszuleitungen 7, 7a sowie die Öffnungen 5, 5a, 5b, 5c befinden sich an dem, dem Stempelboden 3 zugewandten Ende des Stempels 2.

Der Stempel 2 steckt in einer in der bevorzugten Ausführungsform ebenfalls massiv ausgebildeten Hülse 8, die den Stempel in einer als Führung 10 dienenden Bohrung aufnimmt, in der der Stempel 2 vor und zurück geschoben werden kann.

Die Hülse 8 besitzt weitere Bohrungen, welche die Zuleitungen 12, 12a für Kühlmittel darstellen und die so geführt sind, dass deren Querschnitte mit den Öffnungen 5, 5c der Kühlmittelzuleitungen 6, 6a zur Deckung kommen, wenn der Stempel 2 in die Hülse 8 hineingeschoben wird. Die Zuleitungen 12, 12a für Kühlmittel und die Kühlmittelzuleitungen 6, 6a werden in der Beschreibung zur konstruktiven Unterscheidbarkeit terminologisch unterschiedlich benannt, jedoch handelt es sich bei beiden Zuleitungen funktionell um Kühlmittelzuleitungen.

Für den Fall, dass die erfindungsgemäße Probeentnahmevorrichtung dazu verwendet werden soll, die Reaktorflüssigkeit mit einem Stoff bei der Entnahme aus dem Bioreaktor oder aus einem Reaktor zu mischen, sind die Zuleitungen 6, 6a,.... und die Zuleitungen 12, 12a,.... als normale Zuleitungen zu verstehen, deren Funktion sich nicht auf die Zuleitung eines Kühlmittels richtet. Dann dient die Vorrichtung der schnellen Vermischung mit einem anderen Stoff, der vorzugsweise eine andere Flüssigkeit darstellt. Die Beschreibung der erfindungsgemäßen Vorrichtung trifft auch auf diese Ausführungsform zu.

Der Stempel 2 setzt sich zwischen den Öffnungen 5, 5c der Kühlmittelzuleitung 6, 6a und dem Stempelboden 3 noch so weit fort, dass der Abstand zwischen Stempelboden 3 und der Öffnung 5, 5c noch mindestens dem Durchmesser der Öffnung 5, 5c entspricht, so dass die Zuleitungen 12, 12a durch herausziehen des Stempels 2 verschlossen werden können. Dies ist in Figur 2 zu sehen. Der eingezogene Stempelboden 3 bildet dann einen abdichtenden Verschluss gegenüber dem Reaktorinnenvolumen.

Die Hülse 8 schließt an der Seite, an der der Stempelboden 3 ist, mit der Wand 9 ab. Die Wände 11, 11a, 11b, .... stellen ebenfalls den Abschluss der massiven Hülse 8 dar und umschließen den Volumenbereich des Stempels 2 bzw. der Führung 10, in den der Stempel 2 hingeführt wird um die Öffnungen 5, 5c mit den Zuleitungen 12, 12a für Kühlmittel zur Deckung zu bringen. Dabei sparen die Wände 11, 11 a, 11 b....den Teil aus, in den die Öffnungen 5b, 5c der Reaktorflüssigkeitszuleitungen 7, 7a münden, um Reaktorflüssigkeit aufzunehmen. Es ist auch möglich, dass die Wände 11, 11c eine einzige Wand ausbilden, die dann den Stempelboden 3 von dem Reaktorinnenraum trennt.

Die Kühlmittelzuleitungen 6, 6a und der Reaktorflüssigkeitszuleitungen 7, 7a können gleiche oder verschiedene Querschnitte haben. Das Verhältnis der Querschnitte von Kühlmittelzuleitungen 6, 6a und Reaktorflüssigkeitszuleitungen 7, 7a hängt von der gewünschten Kühlleistung aber auch von dem gewählten Kühlmittel und der Temperatur des Kühlmittels ab.

In der in den Figuren dargestellten Probeentnahmevorrichtung 1 sind jeweils 2 Kühlmittelzuleitungen 6, 6a, Reaktorflüssigkeitszuleitungen 7, 7a und Zuleitungen 12, 12a für Kühlmittel dargestellt, die kreuzförmig zusammenlaufen.

Alternativ können jedoch auch jeweils 1, 2, 3 oder 4 oder mehr Kühlmittelzuleitungen 6, 6a, Reaktorflüssigkeitszuleitungen 7, 7a und Zuleitungen 12, 12a....für Kühlmittel zusammengeführt werden. Die Anzahl der Kühlmittelzuleitungen 6, 6a.... und der Reaktorflüssigkeitszuleitungen 7, 7a .... ist frei wählbar und nur durch die technische Realisierbarkeit beschränkt. Es ist möglich dass 1, 2, 3, 4 oder mehr Reaktorflüssigkeitszuleitungen 7, 7a .... und 1, 2, 3, 4 oder mehr Kühlmittelzuleitungen 6, 6a ..... mit der Mischvorrichtung 4 in Verbindung stehen. Wesentlich ist, dass die Reaktorflüssigkeitszuleitungen 7, 7a und die Kühlmittelzuleitungen 6, 6a die Flüssigkeitsströme aus Kühlmittel und Reaktorflüssigkeit möglicht genau auf einen Punkt zusammenführen. Dazu sind die der Kühlmittelzuleitungen 6, 6a.... und der Reaktorflüssigkeitszuleitungen 7, 7a .... kreuzförmig oder sternförmig angeordnet sein, so dass die Flüssigkeitsströme auf das Zentrum des Kreuzes oder Sterns gerichtet werden. Das hat die Folge, dass im Treffpunkt der Flüssigkeitsströme bereits eine maximale Vermischung zwischen den Flüssigkeiten auftritt. Dabei können Kühlmittelzuleitungen 6, 6a anderen Kühlmittelzuleitungen 6, 6a mit den dazugehörigen Zuleitungen 12, 12a, ... für Kühlmittel in einer bevorzugten Ausführungsform gegenüberliegen. Auch die Reaktorflüssigkeitszuleitungen 7, 7a können in einer bevorzugten Ausführungsform jeweils gegenüberliegen. Das hat den Vorteil, dass die Durchmischung der Kühlmittel- und Reaktorflüssigkeitsströme optimiert ist.

Die Reaktorflüssigkeitszuleitungen 7, 7a und die Kühlmittelzuleitungen 6, 6a spannen vorzugsweise eine Ebene auf. Das hat zur Folge, dass sich keine oder kaum Parallelströme von Kühlmittel und Reaktorflüssigkeit bilden, die in der Mischvorrichtung längere Zeit parallel laufen und daher weniger gut gemischt werden. Treffen die kreuzförmig oder sternförmig angeordneten Reaktorflüssigkeitszuleitungen 7, 7a und Kühlmittelzuleitungen 6, 6a in einem Punkt aufeinander, der sich auf der Ebene befindet, die durch die Reaktorflüssigkeitszuleitungen 7, 7a und Kühlmittelzuleitungen 6, 6a aufgespannt wird, so ist das Mischergebnis am besten. Damit ist auch das Kühlergebnis bezüglich benötigtem Zeitaufwand und Homogenität im Flüssigkeitsgemisch optimal. Besonders bevorzugt ist die Ebene die durch die Reaktorflüssigkeitszuleitungen 7, 7a und Kühlmittelzuleitungen 6, 6a aufgespannt wird, senkrecht zum Verlauf der Mischröhre 1 angeordnet. Damit kann eine Parallelströmung reduziert werden. Es ist auch denkbar, dass die Kühlmittelzuleitungen 6, 6a und die Reaktorflüssigkeitszuleitungen 7, 7a nicht auf einer Ebene liegen.

Die erfindungsgemäße Probeentnahmevorrichtung 1 verfügt weiterhin über Mittel zur Erzeugung eines Druckgradienten zwischen dem Innenraum des Bioreaktors und dem Austritt der Mischvorrichtung 4. Diese Mittel können unterschiedlich ausgestaltet sein und in unterschiedlicher Kombination vorliegen. Vorzugsweise ist am Austrittsende der Mischvorrichtung 4 eine Pumpe angebracht, welche Flüssigkeit aus der Mischvorrichtung 4 absaugt. Es kann jedoch auch alleine oder in Kombination mit der Pumpe eine Beaufschlagung des Innenraumes des Bioreaktors mit Druck erfolgen, so dass Flüssigkeit aus dem Innenraum des Reaktors durch die Mischvorrichtung 4 herausgedrückt wird. Zur Beaufschlagung des Bioreaktors mit Druck können Ventile geeignet sein, welche mit einem unter Druck stehenden Gasvorrat verbunden sind und über die ggf. unter Verwendung eines Pneumostaten ein bestimmter Druck eingestellt werden kann. Der Einsatz einer Absaugpumpe ist jedoch die bevorzugte Ausführungsform, da sie gleichermaßen einen Zug an den Kühlmittelzuleitungen 6, 6a und Reaktorflüssigkeitszuleitungen 7, 7a bewirkt, was eine gleichmäßige Zufuhr des Kühlmittels und der Reaktorflüssigkeit bewirkt.

Um beispielsweise auf sich ändernde Viskositäten der Reaktorflüssigkeit reagieren zu können ist es vorteilhaft, weitere Maßnahmen vorzusehen, die eine Änderung der Mischverhältnisse zwischen den Reaktorflüssigkeitsströmen und den Kühlmittelströmen ermöglichen. Hierzu ist es möglich die Zuleitungen 12, 12a mit Regelventilen und/oder Pumpen auszustatten, welche eine Regulierung des Kühlmittelzustroms ermöglichen.

Die Mischvorrichtung 4 ist vorzugsweise eine Mischröhre, die mit Füllkörpern 13, 13a, 13b.... gefüllt sind, die eine intensive Durchmischung der Flüssigkeitsströme aus den Reaktorflüssigkeitszuleitungen 7, 7a und den Kühlmittelzuleitungen 6, 6a bewirken. Grundsätzlich ist jeder handelsübliche Füllkörper 13 geeignet. Eine besonders gute Durchmischung ist möglich, wenn die Füllkörper 13 die Form von Flächenelementen aufweisen, die an einer Kante entlang des Durchmessers der Mischröhre aufgespannt sind und entlang der Rotationsachse der Mischröhre verdreht sind, so dass sie mit der, der ersten Kante gegenüberliegenden Kante wiederum entlang der Rotationsachse der Mischröhre aufgespannt sind. Der Begriff aufgespannt ist nicht so auszulegen, dass diese Kanten der Füllkörper 13 an ihren Enden mit der Mischröhre fest verbunden sein müssen, vielmehr können Füllkörper 13 dieser Form auch lose in der Mischröhre liegen. Jedoch sollten sie den Querschnitt der Mischröhre im Wesentlichen ausfüllen, damit sie besonders wirkungsvoll sind. Die Torsion dieser Flächenförmigen Füllkörper 13, 13a... kann 180°, 90 ° oder jeder andere beliebige Winkel betragen. Weiterhin ist es vorteilhaft, wenn die Füllköper 13, 13a, 13b, 13c.... mit ihren Kanten zueinander um einen Winkel entlang der Rotationsachse der Mischröhre, beispielsweise 90°, zueinander verdreht angeordnet sind. In diesem Fall ist es vorteilhaft, wenn die Füllkörper 13, 13a, 13b, 13c, ... wenigstens teilweise an der Innenwand der Mischröhre befestigt sind um diese Anordnung zueinander zu fixieren. Es kann jedoch auch jeder andere Handelsübliche Füllkörper 13, 13a... eingesetzt werden.

In einer einfachen Ausführungsform der erfindungsgemäßen Vorrichtung kann auch auf Füllkörper 13, 13a ... verzichtet werden, wenn dann immer noch eine ausreichend gute Durchmischung satt findet. In diesem Sinne werden von den Mischvorrichtungen auch Ausführungsformen ohne Füllkörper 13 umfasst.

Bei der erfindungsgemäßen Probeentnahmevorrichtung ragen die Reaktorflüssigkeitszuleitungen 7, 7a mit ihren offenen Enden vorzugsweise in den Innenraum des Bioreaktors. Das hat den Vorteil, dass die Probeentnahme unmittelbar im Innenraum des Bioreaktors erfolgen kann und der Kühlort so nah wie möglich an der Reaktorflüssigkeit mit den lebenden Zellen ist. Dies hat zur Folge, dass Totvolumina minimiert werden und die Kühlung schnellstmöglich bei der Entnahme erfolgen kann, so dass eine Veränderung der stofflichen Zusammensetzung innerhalb der Zelle, beispielsweise durch sich verändernde Stoffwechselvorgänge minimiert oder sogar ausgeschlossen werden kann. Die Zellen können dann mit der Probe gegenüber dem Zustand während des Reaktorbetriebes weitgehend oder komplett unverändert entnommen werden.

Es ist in einer weniger bevorzugten Ausführungsform auch denkbar, dass die Reaktorflüssigkeitszuleitungen 7, 7a sich außerhalb des Reaktors befinden und durch weitere Leitungen mit dem Innenraum des Reaktors verbunden sind. Die Kühlmittelzuleitungen 6, 6a können sich dann ebenfalls außerhalb des Reaktors befinden. Dann ist die Kühlung wegen der unmittelbaren Reaktornähe immer noch sehr gut, jedoch gegenüber der bevorzugten Ausführungsform verzögert. Bei einer solchen Anordnung können die Reaktorflüssigkeitszuleitungen 7, 7a über Zuleitungen beispielsweise in Form von Schläuchen mit dem Reaktor verbunden sein, aus dem sie gespeist werden. Um sicher zu stellen, dass die Reaktorflüssigkeit bis zum Eintritt in die Reaktorflüssigkeitszuleitung 7, 7a möglich unverändert bezüglich ihrer biochemischen Zusammensetzung ist sollte das Volumen der Zuleitung bzw. der Schläuche möglichst gering sein.

Der Abstand des Punktes, in dem die Kühlmittelflüssigkeit oder Flüssigkeit durch die Leitungen 6, 6a und die Reaktorflüssigkeit durch die Reaktorflüssigkeitszuleitungen 7, 7a zusammentreffen von der Reaktorwand 16 hängt auch von den Strömungsgeschwindigkeiten, der Flüssigkeiten und damit von den Querschnitten der Leitungen 12, 12a, 6, 6a, und 7, 7a , deren Verhältnissen zueinander, der Temperatur des Kühlmittels, der Viskosität der Flüssigkeiten und der bei der Abkühlung gewünschten zu erreichenden Temperatur ab. Praktikabel sind daher 20 cm, 15 cm, 10, cm 5 cm oder 4 cm, 3 cm, 2 cm, 1 cm oder im Millimeterbereich 9 mm, 8 mm, 7 mm, 6 mm, 5 mm, 4 mm, 3 mm, 2 mm, oder 1 mm Abstand des Punktes an dem die Kühlmittel- bzw. Flüssigkeitsströme mit der dem Reaktorflüssigkeitsstrom zusammengeführt werden von der Reaktorwand 16.

Die erfindungsgemäße Probeentnahmevorrichtung kann fester Bestandteil eines Bioreaktors, bzw. eines Reaktors, oder lösbar mit ihm verbunden sein.

In der Ausführungsform, bei der die Probeentnahmevorrichtung 1 lösbar mit dem Reaktor verbunden ist kann diese, wie in den Figuren 6b, 6c, 6d, 7a, 7b dargestellt beispielsweise über ein Befestigungsmittel in Form einer Schraube 15, die in ein Gewinde eingreift, welches in die Oberfläche der Hülse 8 geschnitten ist, lösbar verbunden sein. Der Ring 14 fixiert die Probeentnahmevorrichtung 1 an einer Öffnung der Reaktorwand 16 und die Probeentnahmevorrichtung 1 kann von der Gegenseite beispielsweise durch die Schraube 15 fixiert werden. Alternativ ist es auch denkbar, den Ring als Flanschverbindung auszugestalten, die dann an die Reaktorwand 16 angebracht wird.

Die Hülse und/oder die Mischvorrichtung müssen nicht zwingend einen kreisrunden Querschnitt aufweisen. Es ist auch denkbar dass die Querschnitte von der Kreisform abweichen, beispielsweise in Form anderer Rundungen oder in Form von polygonalen Querschnitten.

In einer alternativen Ausführungsform kann die Erfindungsgemäße Vorrichtung auch aus nicht-massiven Teilen gefertigt sein.

In dieser Ausführungsform ist die Hülse 8 als Hohlkörper ausgebildet, welcher die Mischvorrichtung 4 aufnimmt. Eine Führung 10 kann dann in Form von in den Figuren nicht dargestellten Stegen bestehen, die von der Innenwand der Hülse 8 an die Wand der Mischvorrichtung 4 herangeführt sind und diese stützen. Bei dieser Ausführungsform ist dann die Wand 11, 11 a, 11 b, 11 c so ausgestaltet, dass sie eine Wandung ausbildet, die den eingeführten Stempel 2 umschließt, so dass hier ebenfalls eine Führung und Fixierung möglich ist. Weiterhin kann die Mischvorrichtung 4 ihrerseits aus einer dünnen Röhre bestehen, von der die Kühlmittelzuleitungen 6, 6a und die Reaktorflüssigkeitszuleitungen 7, 7a herausragen.

Die Kühlmittelzuleitungen 6, 6a und die Reaktorflüssigkeitszuleitungen 7, 7a ragen dann am Ende der Mischvorrichtung 4 heraus und werden in die Wandung eingeführt, welche einen Zutritt der Kühlmittelzuleitungen 6, 6a zu der Zuleitung 12, 12a ermöglicht. Die Zuleitung kann dann als Rohr ausgebildet sein, welches an der Wandung befestigt ist, die als Führung 10 dient. Vorzugsweise ist die Mischvorrichtung 4 mit dem Kühlmittelzuleitungen 6, 6a und den Reaktorflüssigkeitszuleitungen 7, 7a in eine hohle Röhre eingelagert mit der die Reaktorflüssigkeitszuleitungen 7, 7a und die Kühlmittelzuleitungen 6, 6a über die Öffnungen 5, 5a, 5b, 5c verbunden sind.

Mit dem erfindungsgemäßen Verfahren kann eine Reaktorflüssigkeitsprobe innerhalb von maximal 200 ms in jedem Punkt des Probevolumens auf mindestens -20°C abgekühlt werden. Die Kühlung ist so schnell, dass die biochemischen Reaktionen, die sich im Inneren einer Zelle, die sich in der Reaktorflüssigkeit befindet, gequencht werden, so dass sich die Stoffkonzentrationen im Inneren der Zelle und in der entnommenen Reaktorflüssigkeit nach der Entnahme nicht wesentlich verändern oder im Idealfall unverändert bleiben.

Bei dem erfindungsgemäßen Verfahren wird ein Teil der Reaktorflüssigkeit, die Zellen enthält, in Form eines Reaktorflüssigkeitsstroms einem Kühlmittelstrom zugeführt.

In einer einfachen Ausführungsform wird lediglich ein Reaktorflüssigkeitsstrom mit einem Kühlmittelstrom zusammengeführt.

In einer bevorzugten Ausführungsform werden mindestens zwei Reaktorflüssigkeitsströme mit mindestens einem Kühlmittelflüssigkeitsstrom zusammengeführt. Besonders bevorzugt werden mindestens zwei Reaktorflüssigkeitsströme mit mindestens 2 Kühlmittelströmen zusammengeführt.

Vorzugsweise werden Reaktorflüssigkeitsströme und Kühlmittelströme gleicher Anzahl zusammengeführt. Beispielsweise können 2 Reaktorflüssigkeitsströme und 2 Kühlmittelströme, 3 Reaktorflüssigkeitsströme und 3 Kühlmittelströme, 4 Reaktorflüssigkeitsströme und 4 Kühlmittelströme zusammengeführt werden. Die Anzahl der zusammengeführten Reaktorflüssigkeitsströme und Kühlmittelströme ist erfindungsgemäß nicht nach oberen Werten beschränkt, so dass ihre Anzahl grundsätzlich nach oberen Werten offen ist, jedoch ist eine Beschränkung lediglich durch bauliche oder praktische Schranken gegeben.

In der Ausführungsform bei der mehr als ein Reaktorflüssigkeitsstrom mit jeweils einem Kühlmittelstrom zusammengeführt wird ist es bevorzugt, dass die Flüssigkeitsströme kreuz- oder sternförmig zusammengeführt werden.

Weiterhin ist es möglich, dass dabei gleichartige Flüssigkeitsströme frontal aufeinandertreffen.

Weiterhin ist bevorzugt, dass sich Reaktorflüssigkeitsströme und Kühlmittelströme in einer kreuzförmigen oder sternförmigen Anordnung alternieren, so dass ein Reaktorflüssigkeitsströme von zwei Kühlmittelströmen und ein Kühlmittelstrom von zwei Reaktorflüssigkeitsströmen umgeben ist.

Diese Anordnung hat den Vorteil, dass eine maximale Durchmischung von Reaktorflüssigkeitsstrom und Kühlmittelstrom erfolgt.

Idealerweise treffen sich die Reaktorflüssigkeitsströme und Kühlmittelströme in einem Punkt.

Vorzugsweise spannen die zusammengeführten Reaktorflüssigkeitsströme und Kühlmittelströme eine Ebene auf. Dadurch wird die Ausbildung von Parallelströmen stark reduziert oder sogar unterbunden, was ebenfalls eine gute Durchmischung und damit eine gute Kühlung zur Folge hat.

Die zusammengeführten Reaktorflüssigkeitsströme und Kühlmittelströme werden dann von dem die Reaktorflüssigkeit enthaltenen Reaktor abgeleitet.

In einer anderen Ausgestaltung des Verfahrens wird der Reaktorflüssigkeitsstrom oder werden die Reaktorflüssigkeitsströme nach dem oben beschriebenen Verfahren mit einer anderen Flüssigkeit zusammengeführt, die keine Kühlflüssigkeit ist. Dies dient dann zur schnellen Durchmischung der Reaktorflüssigkeit mit einem anderen Stoff. An Stelle der schnellen Kühlung tritt dann die schnelle Durchmischung. Die Verfahrensweise die im Übrigen beschrieben ist, gilt dann auch für diese Ausführungsform.

Die Ableitung erfolgt vorzugsweise durch eine Mischvorrichtung, die besonders vorteilhaft aus einer Mischröhre besteht, die mit Füllkörpern 13, 13a... beschickt ist. Damit wird eine besonders gute Mischung herbeigeführt, die eine besonders effektive Kühlung bewirkt.

In einer bevorzugten Ausführungsform wird der aus dem Zusammentreffen von Reaktorflüssigkeitsströmen und Kühlmittelströmen resultierende Flüssigkeitsstrom senkrecht zu der von dem Reaktorflüssigkeitsströmen und Kühlmittelströmen aufgespannten Ebene abgeleitet.

Die Ableitung erfolgt dann vorzugsweise durch eine Mischvorrichtung.

In einer bevorzugten Ausführungsform wird der Reaktorflüssigkeitsstrom bzw. die Reaktorflüssigkeitsströme und der Kühlmittelstrom bzw. die Kühlmittelströme so nah wie möglich an der Reaktorwand 16 zusammengeführt, damit die Kühlung an einem Ort erfolgt, an dem sich die biochemische Zusammensetzung der Zellen und /oder der Reaktorflüssigkeit noch nicht verändert hat. Das hat zur Folge, dass Totvolumina minimiert werden und die Kühlung schnellstmöglich erfolgt.

Vorzugsweise werden die Reaktorflüssigkeitsströme und die Kühlmittelflüssigkeitsströme im Innenraum des Reaktors zusammengeführt. Zu diesem Zweck können Zuleitungen für Kühlmittel 12, 12a, mit Kühlmittelzuleitungen 6, 6a die einen Zugang zu einer Ableitung haben in das Reaktorinnere eingebracht werden, die mit Reaktorflüssigkeitszuleitungen 7, 7a derart in Verbindung stehen, dass Reaktorflüssigkeitsströme und Kühlmittelströme zusammentreffen. Es ist aber auch möglich, die Reaktorflüssigkeitsströme und die Kühlmittelströme in der Reaktorwand 16 oder außerhalb des Reaktors zusammenzuführen.

Dann ist bevorzugt, dass der Abstand des Punktes in dem die Flüssigkeitsströme zusammengeführt werden zu der Reaktorwand 16 gering ist. Je dichter sich dieser Punkt an der Reaktorwand 16 befindet, desto weniger hat sich die chemische bzw. biochemische Zusammensetzung der Reaktorflüssigkeit, deren Zustand zu konservieren ist, vor der Kühlung verändert.

Welchen Abstand der Punkt, in dem die Kühlmittel- bzw. Stoffströme und die Reaktorflüssigkeitsströme von der Reaktorwand innerhalb oder außerhalb des Reaktors haben, hängt von dem zu lösenden Problem ab, nämlich, wie schnell eine Kühlung oder Mischung erfolgen soll. Der Abstand dieses Punktes von der Reaktorwand hängt auch von den Strömungsgeschwindigkeiten, der Flüssigkeiten und damit von den Querschnitten der Leitungen 12, 12a, 6, 6a, und 7, 7a, deren Verhältnissen zueinander, der Temperatur des Kühlmittels, der Viskosität der Flüssigkeiten und der bei der Abkühlung gewünschten zu erreichenden Temperatur ab. Praktikabel sind daher 20 cm, 15 cm, 10, cm 5 cm oder 4 cm, 3 cm, 2 cm, 1 cm oder im Millimeterbereich 9 mm, 8 mm, 7 mm, 6 mm, 5 mm, 4 mm, 3 mm, 2 mm, oder 1 mm Abstand des Punktes an dem die Kühlmittel- bzw. Flüssigkeitsströme mit der dem Reaktorflüssigkeitsstrom zusammengeführt werden von der Reaktorwand 16.

Als Kühlmittel kommen vorzugsweise Flüssigkeiten in Frage, die die Zellen nicht schädigen. Insbesondere ist es vorteilhaft, wenn ein Kühlmittel eingesetzt wird, welches die Zellmembran nicht schädigt bzw. eine Lyse der Zellen verhindert, damit die intrazelluläre und extrazelluläre Konzentration von Stoffen separat bestimmt werden kann.

Beispielhaft können Methanol, Glycerin- Salz-Lösungen oder Methanol-Wassergemische eingesetzt werden, beispielsweise Lösungen aus 60 % Methanol und 40 % Wasser, 40 % Glycerin/NaCl-Lösung und 60 % Wasser, 60 % Glycerin-NaCl-Lösung und 40 % Wasser.

Die Kühlflüssigkeiten können auf eine Temperatur von -20°C bis -30°C, vorzugsweise -30°C bis -40°C, -40°C bis -60°C besonders bevorzugt -60°C bis -80°C temperiert sein, um ein besonders gutes Kühlergebnis zu erzielen. Je niedriger die gewählte Temperatur ist, desto niedriger ist das benötigte Kühlmittelvolumen um das gewünschte Kühlergebnis zu erhalten und umso weniger wird die Reaktorflüssigkeit verdünnt.

Die Kühlmittelströme werden vorzugsweise durch eine Pumpe in den Reaktorflüssigkeitsstrom eingespeist.

Alternativ oder ergänzend kann das resultierende Reaktorflüssigkeits- und Kühlmittelgemisch durch eine Pumpe angezogen werden.

Dieser Abzug erfolgt dann vorteilhaft durch die Mischvorrichtung als zwischengeschaltetem Element.

Es ist auch möglich, den Reaktorinnenraum mit Druck zu beaufschlagen, damit der Reaktorflüssigkeitsstrom mit dem Kühlmittelstrom zusammengeführt wird.

Verändert sich während des Prozesses im Reaktor beispielsweise die Viskosität der Reaktorflüssigkeit, so können die Mischverhältnisse zwischen den Reaktorflüssigkeitsströmen und den Kühlmittelströmen variiert werden. Diese Variation kann durch Pumpen und Ventile die den Zustrom der Kühlflüssigkeit und/oder den Abstrom des Kühlmittel- Reaktorflüssigkeitsgemisches beeinflussen, bewirkt werden.

In der Wahl der zu untersuchenden biologischen Zell oder Stoffwechselprozesse ist der Fachmann in seiner Wahl frei.

Die mit dem erfindungsgemäßen Verfahren erhaltenen, gekühlten Proben können entweder sofort zu Analysezwecken eingesetzt werden oder durch Kühlung in Trockeneis oder flüssigem Stickstoff oder in einer Kühltruhe, die beispielsweise bis zu -80°C kühlt längere Zeit aufbewahrt werden.

### Beispiel:

Im vorliegenden Beispiel soll, die Stoffwechselantwort eines Mikroorganismus auf einen Nährstoffimpuls gemessen werden. Hierfür wird der Organismus zunächst unter Nährstoffmangel im Biorektor kultiviert. Im stationären Fließgleichgewicht wird ein Nährstoffpuls zugegeben. Dabei soll eine maximale Vermischung im Reaktor herbeigeführt werden, damit sich die zugegebenen Nährstoffe möglicht schnell homogen im Reaktorflüssigkeitsvolumen verteilen. Zeitgleich wird ein erfindungsgemäßes Probenahmeventil mit integrierter Kühlung geöffnet. Von diesem Zeitpunkt an wird durch Pumpen in einem vorgegebenen Verhältnis Kühlflüssigkeit zugeführt und gekühlte Biomasse abgezogen. Das Verhältnis der durch die Pumpen geförderten Stoffströme wird vorab ggf. durch Computersimulation so festgelegt, dass die geforderten Kühlspezifikationen für das konkrete System eingehalten werden. Die gekühlten Proben werden in Zeitintervallen von 200 Millisekunden fraktioniert und vor der weiteren Verwendung zu Analysezwecken eingefroren.

**Zeitverzögerung für den Mischvorgang in Abhängigkeit von der Entfernung des Punktes in dem die Kühlmittelflüssigkeit und die Reaktorflüssigkeit zusammentreffen vom Reaktor:**

Durch die Verwendung des Mischsystems getrennt vom Bioreaktor als Mischstrecke kommt es zu einer Verzögerung des Mischvorgangs. Diese Verzögerung soll im Folgenden in Abhängigkeit von der Entfernung des Punktes in dem die Kühlmittelflüssigkeit und die Reaktorflüssigkeit zusammentreffen vom Bioreaktor aufgeführt werden:

| **Volumenstrom Kulturflüssigkeit** *[ml*/*s]* | **Querschnitt des Kulturflüssigkeitseinlasses** *[cm²]* | **Entfernung der Mischstrecke vom Reaktor** *[cm]* | **Resultierende Zeitverzögerung** *[s]* |
|---|---|---|---|
| 12,45 | 0,283 | **5** | **0,114** |
| 12,45 | 0,283 | **10** | **0,227** |
| 12,45 | 0,283 | **20** | **0,455** |
| 12,45 | 0,283 | **30** | **0,682** |
| 12,45 | 0,283 | **50** | **1,137** |
| 12,45 | 0,283 | **100** | **2,273** |
| 12,45 | 0,283 | **150** | **3,41** |

## Patentansprüche

1. Probeentnahmevorrichtung umfassend eine Zufuhr für sich in einem Bioreaktor befindliche Lösung sowie eine Zufuhr für eine weitere Flüssigkeit,
bei der,
mindestens eine Reaktorflüssigkeitszuleitung (7, 7a) mit mindestens einer Kühlmittelzuleitung (6, 6a), in einem Punkt zusammentreffen, an den eine Mischvorrichtung (4) angeschlossen ist,
**dadurch gekennzeichnet,**
**dass** die Reaktorflüssigkeitszuleitung (7, 7a) und die Kühlmittelzuleitung (6, 6a) kreuz- oder sternförmig zusammentreffen.

2. Probeentnahmevorrichtung nach Anspruch 1,
**dadurch gekennzeichnet,**
**dass** an die Mischvorrichtung (4) ein Mittel zum Absaugen von Flüssigkeiten angeschlossen ist.

3. Probeentnahmevorrichtung nach Anspruch 1 oder 2,
**dadurch gekennzeichnet,**
**dass** die Reaktorflüssigkeitszuleitung (7, 7a) und die Kühlmittelzuleitung (6, 6a) in einem Punkt zusammentreffen und in einem Radius um den Punkt in dem die Flüssigkeiten zusammengeführt werden, eine Ebene aufspannen.

4. Probeentnahmevorrichtung nach einem der Ansprüche 2 oder 3,
**dadurch gekennzeichnet,**
**dass** die Mischvorrichtung (4) eine Röhre ist, die mit Füllkörpern (13, 13a) gefüllt ist, die eine Ableitung der zu mischenden Flüssigkeitsströme ermöglicht.

5. Probeentnahmevorrichtung nach einem der Ansprüche 2 bis 4,
**dadurch gekennzeichnet,**
**dass** die Röhre, welche die Füllkörper (13, 13a) enthält, senkrecht auf die Ebene trifft, welche durch die Reaktorflüssigkeitszuleitung (7, 7a) und Kühlmittelzuleitung (6, 6a) aufgespannt wird.

6. Probeentnahmevorrichtung nach Anspruch 5,
**dadurch gekennzeichnet,**
**dass** die Füllkörper (13, 13a) Flächenelemente sind, welche an einer Kante entlang des Durchmessers der Röhre aufgespannt sind und mit der gegenüberliegenden Kante um 180 ° entlang der Durchmessers der Röhre aufgespannt sind.

7. Probeentnahmevorrichtung nach Anspruch 6,
**dadurch gekennzeichnet,**
**dass** die Kanten verschiedener Flächenelemente bezüglich der Rotationsachse der Röhre um 90° zueinander verdreht angeordnet sind.

8. Probeentnahmevorrichtung nach einem der Ansprüche 1 bis 7,
**dadurch gekennzeichnet,**
**dass** die Kühlmittelzuleitungen (6, 6a) an Zuleitungen (12, 12a) angeschlossen sind, welche eine Versorgung mit dem Kühlmittel ermöglichen.

9. Probeentnahmevorrichtung nach einem der Ansprüche 1 bis 8,
**dadurch gekennzeichnet,**
**dass** die Mischvorrichtung (4), die Reaktorflüssigkeitszufuhr (7, 7a) und die Kühlmittelzufuhr (6, 6a) eine Einheit darstellen, welche als Stempel (2) an die Wand eines Reaktors herangeführt werden kann, wobei die Reaktorflüssigkeitszufuhr (7, 7a) mit dem Flüssigkeitsvolumen des Reaktors in Kontakt kommt und die Querschnitte der Kühlmittelzufuhr (6, 6a) mit dem Querschnitt der Leitung für die Zufuhr des Kühlmittels (12, 12a) zur Deckung kommt.

10. Probeentnahmevorrichtung nach Anspruch 9,
**dadurch gekennzeichnet,**
**dass** die Ebene, die durch die Kühlmittelzuleitungen (6, 6a) und die Reaktorflüssigkeitszuleitungen (7, 7a) aufgespannt wird, in den Innenbereich des Reaktors hineinragt, so dass der Volumenstrom aus Reaktorflüssigkeit ohne Totvolumen aus dem Reaktorinneren bezogen werden kann.

11. Probeentnahmevorrichtung nach einem der Ansprüche 1 bis 10,
**dadurch gekennzeichnet,**
**dass** sie eine Hülse (8) umfasst, welche eine axiale Führung (10) zum Einführen eines Stempels (2) aufweist, einen Stempel (2), der in die Führung (10) eingeführt werden kann, und der die Mischvorrichtung (4) beinhaltet, die in eine Röhre aufgenommen ist,
wobei der Stempel (2) an dem Ende, welches der Reaktorwand (16) zugewandt is, einen Stempelboden (3) besitzt, der den Stempel (2) gegenüber seinem Inneren abschließt und dass sich an dem dem Stempelboden (3) zugewandten Ende des Stempels (2) mindestens eine Reaktorflüssigkeitszuleitung (7, 7a) und mindestens eine Kühlmittelzuleitung (6, 6a) befindet, die eine Verbindung zwischen der Röhre und dem Außenradius des Stempels darstellt und dass die Hülse (8) mindestens eine Zuleitung (12, 12a) umfasst, welche in die Kühlmittelzuleitung (6, 6a) münden, wobei die Führung (10) an Öffnungen, 5 a, 5b, besitzt, mit denen die Reaktorflüssigkeitszuleitungen (7, 7a) zur Deckung kommen, wenn der Stempel 2 in die Führung eingeschoben ist.

12. Verfahren zur Probeentnahme aus einem Bioreaktor, bei dem eine Kühlung oder Durchmischung der Probe mit einer anderen Flüssigkeit aus dem Bioreaktor vorgenommen wird,
bei dem
mindestens ein Reaktorflüssigkeitsstrom mit mindestens einem Kühlmittelstrom oder anderem Flüssigkeitsstrom in einen Punkt zusammengeführt wird und durch eine Mischvorrichtung gefördert wird
**dadurch gekennzeichnet,**
**dass** die Reaktorflüssigkeitsströme und die Kühlmittelströme kreuz- oder sternförmig zusammengeführt werden.

13. Verfahren zur Probeentnahme nach Anspruch 12,
**dadurch gekennzeichnet,**
**dass** die Reaktorflüssigkeitsströme und die Kühlmittelströme die in einem Punkt zusammengeführt werden eine Ebene aufspannen.

14. Verfahren nach einem der Ansprüche 12 oder 13,
**dadurch gekennzeichnet,**
**dass** die zusammengeführten Kühlmittelströme und Reaktorflüssigkeitsströme über eine Mischvorrichtung abgesaugt werden.

15. Verfahren nach einem der Ansprüche 12 bis 14,
**dadurch gekennzeichnet,**
**dass** die Flüssigkeitsströme für die Reaktorflüssigkeit und die Kühlflüssigkeit im Innenbereich eines Bioreaktors zusammengeführt werden.

## Claims

1. Sampling device comprising a feed for a solution contained within a bioreactor as well as a feed for a further fluid, wherein at least one reactor fluid feed line (7, 7a) converges with at least one coolant feed line (6, 6a) at a point at which a mixing device (4) is connected, **characterised in that** the reactor fluid feed line (7, 7a) and the coolant feed line (6, 6a) converge in the form of a cross or star.

2. Sampling device according to claim 1, **characterised in that** a means for extracting fluids by suction is connected to the mixing device (4).

3. Sampling device according to claim 1 or 2, **characterised in that** the reactor fluid feed line (7, 7a) and the coolant feed line (6, 6a) converge at a point and span a plane in a radius around the point at which the fluids are brought together.

4. Sampling device according to one of the claims 2 or 3, **characterised in that** the mixing device (4) is a tube which is filled with packing elements (13, 13a) which makes it possible to drain off the fluid flows which are to be mixed.

5. Sampling device according to one of the claims 2 to 4, **characterised in that** the tube containing the packing elements (13, 13a) intersects perpendicularly with the plane which is spanned by the reactor fluid feed line (7, 7a) and coolant feed line (6, 6a).

6. Sampling device according to claim 5, **characterised in that** the packing elements (13, 13a) are planar elements which are mounted on one edge along the diameter of the tube, with the opposite edge mounted at 180° along the diameter of the tube.

7. Sampling device according to claim 6, **characterised in that** the edges of different planar elements are arranged rotated at 90° to one another in relation to the axis of rotation of the tube.

8. Sampling device according to one of the claims 1 to 7, **characterised in that** the coolant feed lines (6, 6a) are connected to supply lines (12, 12a) which make it possible to supply the coolant.

9. Sampling device according to one of the claims 1 to 8, **characterised in that** the mixing device (4), the reactor fluid feed line (7, 7a) and the coolant feed line (6, 6a) represent a unit which can be applied to the wall of a reactor as a punch (2), wherein the reactor fluid feed line (7, 7a) comes into contact with the fluid volume of the reactor and the cross sections of the coolant feed (6, 6a) align with the cross section of the line for the supply of the coolant (12, 12a).

10. Sampling device according to claim 9, **characterised in that** the plane which is spanned by the coolant feed lines (6, 6a) and the reactor fluid feed lines (7, 7a) projects into the interior of the reactor, so that the volume flow of reactor fluid can be drawn from the interior of the reactor without any dead volume.

11. Sampling device according to one of the claims 1 to 10, **characterised in that** it comprises a sleeve (8) which possesses an axial guide (10) for introducing a punch (2), a punch (2) which can be inserted in the guide (10), and which contains the mixing device (4) which is accommodated in a tube, wherein the punch (2) possesses a punch base (3) at the end which faces the reactor wall (16) which seals off the punch (2) from its interior and that at least one reactor fluid feed line (7, 7a) and at least one coolant feed line (6, 6a) are located at the end of the punch (2) facing the punch base (3), representing a connection between the tube and the outer radius of the punch and that the sleeve (8) comprises at least one feed line (12, 12a) opening into the coolant feed line (6, 6a), wherein the guide (10) possesses openings 5a, 5b which line up with the reactor fluid feed lines (7, 7a) when the punch 2 is pushed into the guide.

12. Method for taking a sample from a bioreactor, in which a cooling or mixing of the sample with another fluid from the bioreactor is carried out, in which the at least one reactor fluid flow is brought together with at least one coolant flow or other fluid flow at a point and transported through a mixing device **characterised in that** the reactor fluid flows and the coolant flows converge in the form of a cross or star.

13. Method for taking a sample according to claim 12, **characterised in that** the reactor fluid flows and the coolant flows which are brought together at a point span a plane.

14. Method according to one of the claims 12 or 13, **characterised in that** the coolant flows and reactor fluid flows which are brought together are drawn off by suction via a mixing device.

15. Method according to one of the claims 12 to 14, **characterised in that** the fluid flows for the reactor fluid and the coolant fluid can be brought together in the interior region of a bioreactor.

## Revendications

1. Dispositif de prélèvement d'échantillons comprenant une amenée pour une solution se trouvant dans un bioréacteur ainsi qu'une amenée pour un autre liquide,
dans lequel
au moins une conduite d'arrivée de liquide de réacteur (7, 7a) et au moins une conduite d'arrivée d'agent de refroidissement (6, 6a) se rencontrent en un point, auquel est raccordé un dispositif de mélange (4),
**caractérisé en ce**
**que** la conduite d'arrivée de liquide de réacteur (7, 7a) et la conduite d'arrivée d'agent de refroidissement (6, 6a) se rencontrent de manière à former une croix ou une étoile.

2. Dispositif de prélèvement d'échantillons selon la revendication 1,
**caractérisé en ce**
**qu'**un moyen servant à aspirer des liquides est raccordé au dispositif de mélange (4).

3. Dispositif de prélèvement d'échantillons selon la revendication 1 ou 2,
**caractérisé en ce**
**que** la conduite d'arrivée de liquide de réacteur (7, 7a) et la conduite d'arrivée d'agent de refroidissement (6, 6a) se rencontrent en un point et forment un plan selon un rayon autour du point, au niveau duquel les liquides se rassemblent.

4. Dispositif de prélèvement d'échantillons selon l'une quelconque des revendications 2 ou 3,
**caractérisé en ce**
**que** le dispositif de mélange (4) est un tube, qui est rempli de corps de remplissage (13, 13a) et qui permet une déviation des flux de liquide devant être mélangés.

5. Dispositif de prélèvement d'échantillons selon l'une quelconque des revendications 2 à 4,
**caractérisé en ce**
**que** le tube, qui contient les corps de remplissage (13, 13a) rencontre verticalement le plan qui est formé par la conduite d'arrivée de liquide de réacteur (7, 7a) et par la conduite d'arrivée d'agent de refroidissement (6, 6a) .

6. Dispositif de prélèvement d'échantillons selon la revendication 5,
**caractérisé en ce**
**que** les corps de remplissage (13, 13a) sont des éléments plats, qui s'étirent au niveau d'une arête le long du diamètre du tube et qui s'étirent avec l'arête opposée selon un angle de 180° le long du diamètre du tube.

7. Dispositif de prélèvement d'échantillons selon la revendication 6,
**caractérisé en ce**
**que** les arêtes des divers éléments plats sont disposées de manière pivotée les unes par rapport aux autres de 90° par rapport à l'axe de rotation du tube.

8. Dispositif de prélèvement d'échantillons selon l'une quelconque des revendications 1 à 7,
**caractérisé en ce**
**que** les conduites d'arrivée d'agent de refroidissement (6, 6a) sont raccordées aux conduites d'arrivée (12, 12a), lesquelles permettent une alimentation en agent de refroidissement.

9. Dispositif de prélèvement d'échantillons selon l'une quelconque des revendications 1 à 8,
**caractérisé en ce**
**que** le dispositif de mélange (4), l'amenée de liquide de réacteur (7, 7a) et l'amenée d'agent de refroidissement (6, 6a) constituent une unité, qui peut être amenée contre la paroi d'un réacteur en tant que poinçon (2), sachant que l'amenée de liquide de réacteur (7, 7a) vient en contact avec le volume de liquide du réacteur et que les sections transversales de l'amenée d'agent de refroidissement (6, 6a) recouvrent la section transversale de la conduite pour l'amenée d'agent de refroidissement (12, 12a).

10. Dispositif de prélèvement d'échantillons selon la revendication 9,
**caractérisé en ce**
**que** le plan formé par les conduites d'arrivée d'agent de refroidissement (6, 6a) et les conduites d'arrivée du liquide de réacteur (7, 7a) fait saillie dans la zone intérieure du réacteur de sorte que le flux volumique de liquide de réacteur peut être fourni sans volume mort depuis l'intérieur du réacteur.

11. Dispositif de prélèvement d'échantillons selon l'une quelconque des revendications 1 à 10,
**caractérisé en ce**
**qu'**il comprend une douille (8), qui présente un guidage (10) axial servant à introduire un poinçon (2), un poinçon (2), qui peut être introduit dans le guidage (10) et qui contient le dispositif de mélange (4) logé dans un tube, sachant que le poinçon (2) comporte, au niveau de l'extrémité tournée vers la paroi de réacteur (16) un fond de poinçon (3), qui se trouve dans le prolongement du poinçon (2) par rapport à l'intérieur de ce dernier, et en ce qu'au moins une conduite d'arrivée de liquide de réacteur (7, 7a) et au moins une conduite d'arrivée d'agent de refroidissement (6, 6a) se trouvent au niveau de l'extrémité du poinçon (2) tournée vers le fond de poinçon (3), lesquelles constituent une liaison entre le tube et le rayon extérieur du poinçon, et en ce que la douille (8) comprend une conduite d'arrivée (12, 12a), qui débouche dans la conduite d'arrivée d'agent de refroidissement (6, 6a), sachant que le guidage (10) comporte des orifices (5a, 5b), que les conduites d'arrivée de liquide de réacteur (7, 7a) recouvrent, lorsque le poinçon (2) est inséré par coulissement dans le guidage.

12. Procédé servant à prélever des échantillons d'un bioréacteur, dans le cadre duquel on procède à un refroidissement ou à un mélange de l'échantillon avec un autre liquide provenant du bioréacteur,
dans le cadre duquel
au moins un flux de liquide de réacteur est regroupé avec au moins un flux d'agent de refroidissement ou avec un autre flux de liquide en un point et est transporté par un dispositif de mélange,
**caractérisé en ce**
**que** les flux de liquide de réacteur et les flux d'agent de refroidissement sont rassemblés de manière à former une croix ou une étoile.

13. Procédé servant au prélèvement d'échantillons selon la revendication 12,
**caractérisé en ce**
**que** les flux de liquide de réacteur et les flux d'agent de refroidissement sont rassemblés en un point et forment un plan.

14. Procédé selon l'une quelconque des revendications 12 ou 13,
**caractérisé en ce**
**que** les flux d'agent de refroidissement et les flux de liquide de réacteur rassemblés sont aspirés par l'intermédiaire d'un dispositif de mélange.

15. Procédé selon l'une quelconque des revendications 12 à 14,
**caractérisé en ce**
**que** les flux de liquide pour le liquide de réacteur et le liquide de refroidissement sont rassemblés dans une zone intérieure d'un bioréacteur.
